# FASCICULE DE BREVET EUROPEEN

(11) **EP 1 009 376 B1**
(45) Date de publication et mention de la délivrance du brevet: **05.06.2002**
(21) Numéro de dépôt: 98903094.5
(22) Date de dépôt: 20.01.1998
(51) Int. Cl.: A61K 7/48, A61K 7/06, A61K 7/04

(54) **PRODUITS COSMETIQUES OU DERMO-PHARMACEUTIQUES RESPECTANT L'ECOLOGIE CUTANEE**
HAUTFREUNDLICHE KOSMETISCHE ODER DERMATOLOGISCHE ZUSAMMENSETZUNG
COSMETIC OR DERMO-PHAMACEUTICAL PRODUCTS COMPATIBLE WITH CUTANEOUS ECOLOGY

(30) Priorité: 21.01.1997 FR 9700803
(43) Date de publication de la demande: 21.06.2000
(73) Titulaire: THOREL, Jean-Noel, F-75014 Paris (FR)
(72) Inventeur: THOREL, Jean-Noel, F-75014 Paris (FR)
(74) Mandataire: Guerre, Dominique
(86) Numéro de dépôt international: FR9800100
(87) Numéro de publication internationale: WO9831337

(56) Documents cités:
- EP-A- 0 210 483
- EP-A- 0 237 398
- EP-A- 0 379 846
- EP-A- 0 475 851
- EP-A- 0 477 833
- EP-A- 0 627 223
- WO-A-89/05629
- WO-A-94/18945
- WO-A-97/25023

## Description

La présente invention concerne la cosmétologie, l'hygiène corporelle, la dermo-thérapeutique par voie topique, et de manière générale tous produits à destination de la peau, ou de parties superficielles du corps humain, environnant l'épiderme, telles que ongles et cheveux.

Traditionnellement, un produit cosmétique ou dermatologique comprend un ou plusieurs principes actifs, et un excipient ou véhicule dans lequel se trouvent distribués ou dispersés le ou les principes actifs, le tout étant formulé pour une application topique, par exemple sous forme de gel ou crème.

Par "principe actif", on entend tout composé, toute composition, ou tout produit exerçant une activité biomécanique, biophysique, physiologique, ou biologique vis-à-vis de la peau, et en particulier de l'épiderme, et ayant toute nature et forme physico-chimique, requises par le traitement ou l'action choisi ou retenu vis-à-vis de la peau.

Le ou les principes actifs retenus, de type chimique, sont dans leur grande majorité étrangers à la peau ; cf EP-0 475 851. Dans certains cas, ces principes actifs sont des produits naturels, c'est-à-dire obtenus à partir de produits ou substances de la nature, par exemple dans le règne végétal ; cf EP-0 477 833. Dans d'autres cas, ces principes actifs sont des produits ou constituants trouvés dans la peau ; cf WO-94/18945 et EP-0 627 223.

Par ailleurs, on connait des produits cosmétiques, ou dermo-pharmaceutiques, ne comprenant pas d'excipient ou véhicule à proprement parler, mais sous forme de complexe, associant divers produits ; cf EP-0 379 846. Dans certains exemples, un ou quelques composants, mais pas la totalité de ces derniers, sont des composants naturels ; cf WO-89/05629.

Aujourd'hui, à la meilleure connaissance du titulaire, personne ne s'est intéressé, à des fins cosmétiques ou thérapeutiques, à la peau, et en particulier à l'épiderme, considérés en tant que milieu ou système écologique, dont il faut respecter l'équilibre, excepté certains travaux concernant uniquement l'écologie de la flore bactérienne cutanée.

Tel est l'objet de la présente invention.

Dans toute sa généralité, l'invention propose une nouvelle génération de produits cosmétiques, ou d'hygiène corporelle, ou dermo-thérapeutique, qui comprennent pour au moins 98 % en poids des constituants biodermiques, choisis chacun pour leur caractère biocompatible et cytocompatible avec les cellules de la peau, et préférentiellement biomimétique avec un composant de la peau, notamment de l'épiderme. Ces constituants biodermiques sont choisis et formulés, d'une part pour exprimer ensemble au moins une bio-activité topique, identique ou différente de la bio-activité d'au moins un constituant biodermique, et d'autre part pour conférer au produit une composition sous forme galénique topique stable, en ce qui concerne le mode d'administration considéré, par exemple gel, lait, crème, lotion, démaquillant, pour un produit cosmétique ou d'hygiène corporelle.

Il résulte de la définition précédente, par différence avec les produits cosmétiques ou dermatologiques traditionnels, qu'il n'est plus possible en particulier de distinguer entre principe(s) actif(s) et excipient ou véhicule. Pratiquement, un produit selon la présente invention se comporte aussi bien comme un ou plusieurs principes actifs, que comme un excipient. Et chaque constituant biodermique doit être vu, aussi bien comme un constituant cutané, et/ou un principe actif, et/ou un agent galénique, par exemple un épaississant ou un tensioactif.

La composition d'un produit selon la présente invention exclut pratiquement tout excipient non biodermique autre que de l'eau ou une phase aqueuse.

Il résulte de la définition précédente qu'un produit selon la présente invention ne peut être assimilé:
- à un ou plusieurs principes actifs biodermiques, y compris biomimétiques, formulés dans une forme galénique stable, avec un excipient ou véhicule traditionnel
- à un complexe de produits naturels, en général sans excipient ou véhicule à proprement parler, puisqu'il comprend pratiquement en totalité des produits naturels particuliers, à savoir des constituants biodermiques.

Selon la définition précédente, se trouve également exclu tout produit limité à un ou plusieurs principes actifs biodermiques, distribués d'une manière ou d'une autre dans de l'eau ou dans une phase aqueuse, majoritaire en poids dans la composition totale du produit; ce produit étant par exemple une lotion obtenue par dispersion d'un glycosaminoglycane dans une phase aqueuse.

Préférentiellement, les constituants biodermiques cyto-compatibles avec la peau représentent la totalité de la composition pondérale du produit considéré.

Préférentiellement, chaque constituant biodermique, biomimétique avec un composant de la peau, a une teneur pondérale dans ledit produit (exprimée en pourcentage pondéral par exemple), différente de celle dudit composant dans la peau.

A titre d'exemple, un produit selon l'invention comprend deux constituants biodermiques et biomimétiques, dont l'un majoritaire est un composant constitutif de l'épiderme, et dont l'autre minoritaire est un nutriment de la peau.

De manière avantageuse, le constituant biodermique est choisi parmi les espèces ou entités biologiques, minérales, organiques, ou biochimiques, composant l'épiderme ou le derme. Un des constituants biodermiques est choisi, à titre d'exemple, parmi les nutriments de la peau.

Un produit conforme à l'invention est biphasique ou monophasique, ou constitué de plusieurs phases émulsionnaires ou non, pour obtenir toute forme topique appropriée, par exemple crème, émulsion, lotion, sérum, lait, gel, fluide, etc...

Par "excipient" ou "véhicule", on entend de manière traditionnelle tout composé ou composition, tel que diluant, dispersant, ou solvant, adapté pour former la forme topique requise, par exemple une crème, un gel, une huile, une lotion, etc... Cette composition est complétée par divers adjuvants bien connus de l'homme de métier, tels qu'agents de mouillage, d'astringence, humidifiants, émolients, etc...

Par "interactive", on entend la propriété selon laquelle la composition d'un produit selon l'invention maintient, sans les inhiber ou les exacerber, tous les équilibres biologiques fonctionnels de la peau, et ceci en plus de la bioactivité topique de ladite composition, résultant de l'activité biologique vis-à-vis de la peau d'un ou plusieurs constituants biodermiques, de manière synergique ou non.

Un produit selon l'invention a donc trois fonctions. Une première fonction consiste à la fois à protéger et maintenir, voire à restaurer les principaux équilibres cutanés, biologiques et physiologiques. Une deuxième fonction consiste à créer une forme galénique stable appropriée (lait, crème, etc...). Une troisième fonction consiste à traiter la peau, en lui apportant un ou plusieurs bénéfices par voie topique, notamment thérapeutiques ou cosmétiques.

Par "forme galénique", on entend toute forme ou présentation, adaptée au mode d'administration retenu, permettant au produit de jouer une activité fonctionnelle vis-à-vis de la peau.

Par "constituant biodermique", on entend tout composant ou produit entrant dans la composition de la peau, notamment de l'épiderme, étant entendu que ce constituant est considéré à l'état isolé, sous une forme identique à sa forme naturelle, ou modifiée par rapport à sa forme naturelle, mais restant cyto-compatible avec la peau, quel que soit son mode d'obtention ou production, en particulier par séparation à partir de produits cutanés vivants, par bio-synthèse, par un procédé biotechnologique, ou encore par recombinaison génétique.

Par "bioactif", on entend le fait que le composant ou constituant considéré présente par lui-même une activité biologique au sein de l'épiderme, ou encore participe à tout processus biologique, tel. que métabolisme, dans l'épiderme.

Par "activité topique", on entend le fait que globalement la base interactive cutanée exhibe ou manifeste, par voie topique, un bénéfice cosmétique ou thérapeutique, au niveau de la peau, par exemple de l'épiderme.

Par "cytocompatible avec la peau", on entend la propriété selon laquelle le constituant biodermique retenu présente une cytotoxicité inférieure à 10 % vis-à-vis d'une culture cellulaire de kératinocytes humains, c'est-à-dire demeure quasiment neutre vis-à-vis de la viabilité cellulaire et la morpho-différenciation des kératinocytes.

Cette cytocompatibilité peut être évaluée par le test de routine suivant .

Des kératinocytes humains normaux issus de chirurgie plastique sont cultivés en condition sub-émergée en milieu défini (MCDB 153) supplémenté avec 10 ng/ml d'un facteur de croissance épidermique, 5 µg/ml d'insuline, 0,1 mM d'éthanolamine, 0,1 mM de phosphoéthanolamine, et 2 % d'acides aminés non essentiels. Ce milieu permet la culture des kératinocytes sans présence, ni de sérum, ni de cellules nourricières vivantes (fibroblastes 3T3) ; sa faible teneur en calcium (0,1 mM) favorise la croissance cellulaire.

Les constituants biodermiques sont évalués quant à leur capacité d'induire des effets cytopathiques sur cultures sub-confluentes. Les temps de contacts sont de 6 heures, 12 heures, 24 heures et 36 heures.

La viabilité cellulaire est mesurée quantitativement par comptage indirect des cellules vivantes après leur marquage par un colorant vital. Le système rouge neutre (hydrochlorure de 3-amino-7-diméthyl-amino-2-méthylphénasine) mesure l'activité de passage du colorant à travers la membrane plasmique et de stockage dans les lysosomes des cellules viables.

L'incorporation totale du rouge neutre est proportionnelle au nombre de cellules vivantes en culture.

Le colorant incorporé est extrait par un solvant acide acétique/éthanol et quantifié par mesure spectrophotométrique. Les résultats sont comparés par rapport aux cultures standards, de manière qualitative, et exprimés en densité optique (DO) et/ou pourcentage de densité optique par rapport au contrôle négatif (culture non traitée).

A la variation près des souches utilisées, un pourcentage supérieur ou égal à 90 % de densité optique pour une culture mise au contact du constituant biodermique testé, par rapport au contrôle négatif, exprime que ledit constituant est cyto-compatible avec la peau.

Par "biomimétique", on entend le fait que le constituant biodermique retenu a la structure de et/ou exerce la fonction ou l'activité biomécanique, biophysique, physiologique, ou biologique de tout composant cutané, c'est-à-dire de tout composant de la peau pouvant être isolé ou séparé par fractionnement du derme et/ou de l'épiderme, ou encore de tout composant dont l'existence peut être caractérisée ou mise en évidence dans le derme et/ou l'épiderme, ou encore de tout composant pouvant être assimilé par la peau et servir le cas échéant de nutriment aux cellules constitutives de cette dernière.

En d'autres termes, la peau, en tant que milieu biologique vivant, ne peut distinguer entre ses propres constituants biodermiques.

Préférentiellement, les constituants biodermiques sont choisis parmi les espèces ou entités, biologiques, minérales, organiques, ou biochimiques, constitutives de la peau, quelle que soit l'origine effective des constituants rapportés sur ou dans la peau.

Les constituants biodermiques peuvent être choisis parmi les molécules constitutives de l'épiderme et du derme. A titre d'exemple, on retiendra :
- différents lipides, fluides ou concrets, comme l'acide oléique, les acides gras essentiels, les mono, di-et tri-glycérides, l'acide linoléique, le squalène, l'acide stéarique, le monostéarate de glycérol,
- les lipides concrets, comme l'acide stéarique, le cholestérol, les céramides, le cholestérol-ester ou sulfate, les diglycérides saturés,
- les acides nucléiques,
- les mucopolysaccarides, comme l'acide hyaluronique
- les collagènes.

Le constituant biodermique peut aussi être choisi parmi les nutriments de la peau, c'est-à-dire les composés ou compositions pouvant être métabolisés par les cellules de la peau.

A titre d'exemple, on citera tous les éléments, ou fragments d'aliments, utiles au métabolisme de la peau, tels qu'acides cutanés, acides gras, vitamines, peptone, caséine, caséïnates, glycoprotéines immuno-globulines, lipides complexes, acides aminés, oligo-éléments, lactosérum.

Si nécessaire, la composition d'un produit cosmétique, ou d'hygiène corporelle, ou dermopharmaceutique, selon la présente invention, peut être complétée, à hauteur d'un maximum de 2 %, par des constituants non biodermiques, mais cytocompatibles avec cette dernière, nécessaires à la mise en forme galénique dudit produit, et/ou pour compléter la bioactivité topique du produit. A titre d'exemple, on citera principalement les parfums, et très accessoirement et exceptionnellement certains adjuvants minoritaires nécessaires au bon maintien de la stabilité et de la pureté du produit cosmétique ou dermo-thérapeutique.

Les constituants biodermiques formant la quasi-totalité, sinon la totalité d'un produit selon l'invention, sont formulés et obtenus selon des techniques traditionnelles, par exemple sous forme biphasique, en étant constitués par une dispersion eau dans huile, ou huile dans eau.

Les tableaux 1 à 3 rassemblent des exemples de constituants biodermiques pour formuler une phase huileuse, et/ou une phase aqueuse, et compléter les formulations en fonction des applications du produit cosmétique. Dans ces tableaux, la colonne de gauche exprime une préconisation, étant entendu que, comme dit précédemment, chacun des constituants biodermiques est par nature cytocompatible avec la peau, et biomimétique avec cette dernière. Les autres colonnes expriment la classe à laquelle appartient ledit composant, selon la classification suivante:

### Classe 1:

Composants substantiellement cytocompatibles et/ou bio-assimilables, présents à l'état constitutionnel dans la peau (eau, acides aminés, oligo-éléments, vitamines...).

### Classe 2:

Macromolécules obtenues par voie biotechnologique, procédé de synthèse ou d'extraction, de composition et de structure identique ou quasi-identique aux constituants de la peau (sel de sodium d'ADN, hyaluronate de sodium...). Ces composés sont cytocompatibles avec la peau, mais ils peuvent présenter des interactions métaboliques dans le cadre de processus biologiques ou physiologiques. Leur action biologique est toutefois comparable à celle des composants présents à l'état naturel dans la peau (bio-mimétisme).

Les composants des classes 1 et 2 sont interactifs, c'est-à-dire jouent à la fois le rôle d'actifs et d'excipients.

### Classe 3:

Composants utiles et assimilables par la peau, si possible d'origine alimentaire ou diététique, ou bien autorisés en matière alimentaire, mais ils doivent être cytocompatibles. Ces composants sont interactifs : ils jouent à la fois un rôle de "dermo-diététique" et d'excipient.

### Classe 4:

Composants inertes, c'est-à-dire qui ne provoquent aucune incidence chimique, biologique ou immunologique avec la peau : composés restant dans les assises cellulaires superficielles de l'épiderme (par exemple, vaseline et autres corps gras d'origine minérale, silicones, dioxyde de titane, oxyde de zinc, mica).

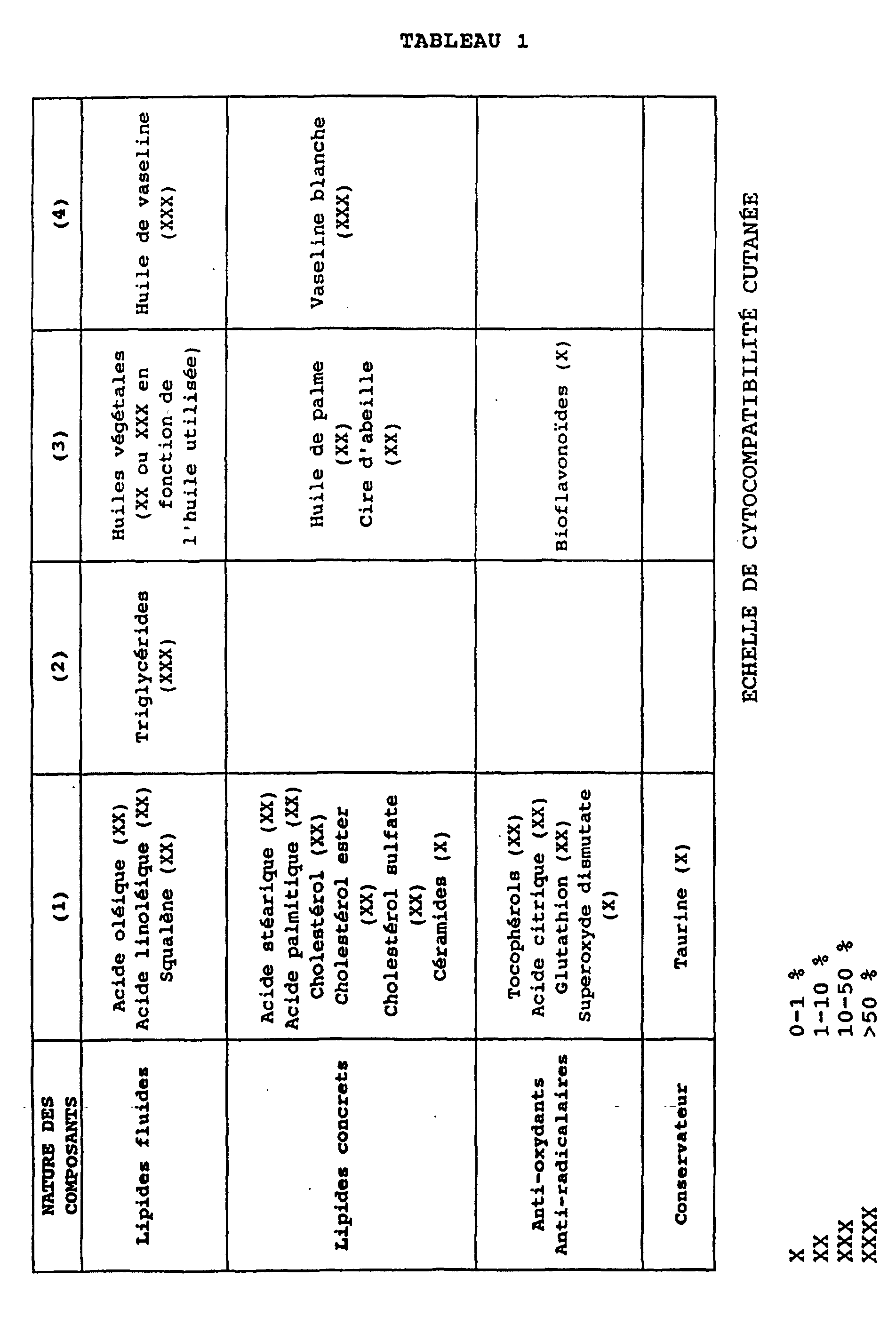

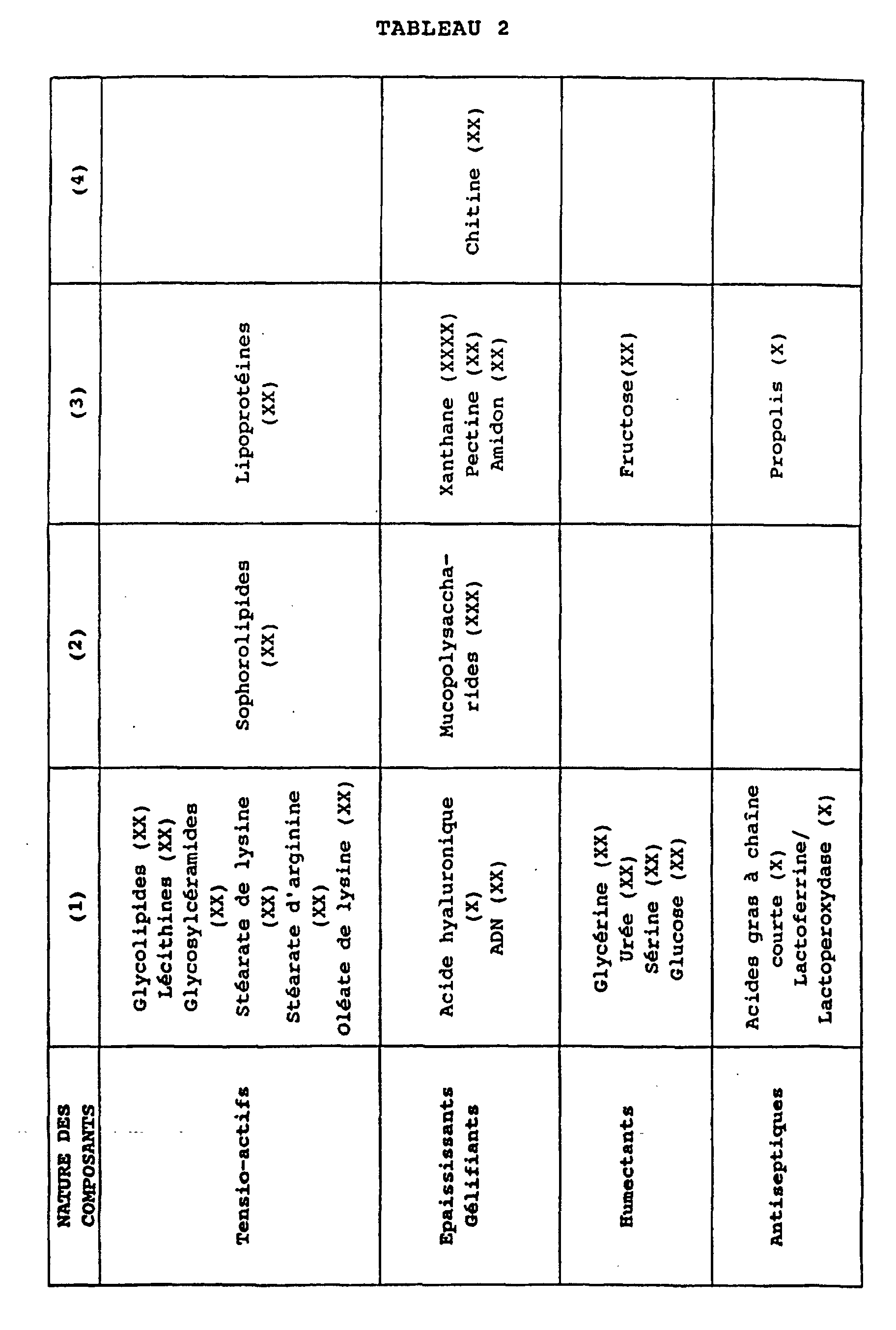

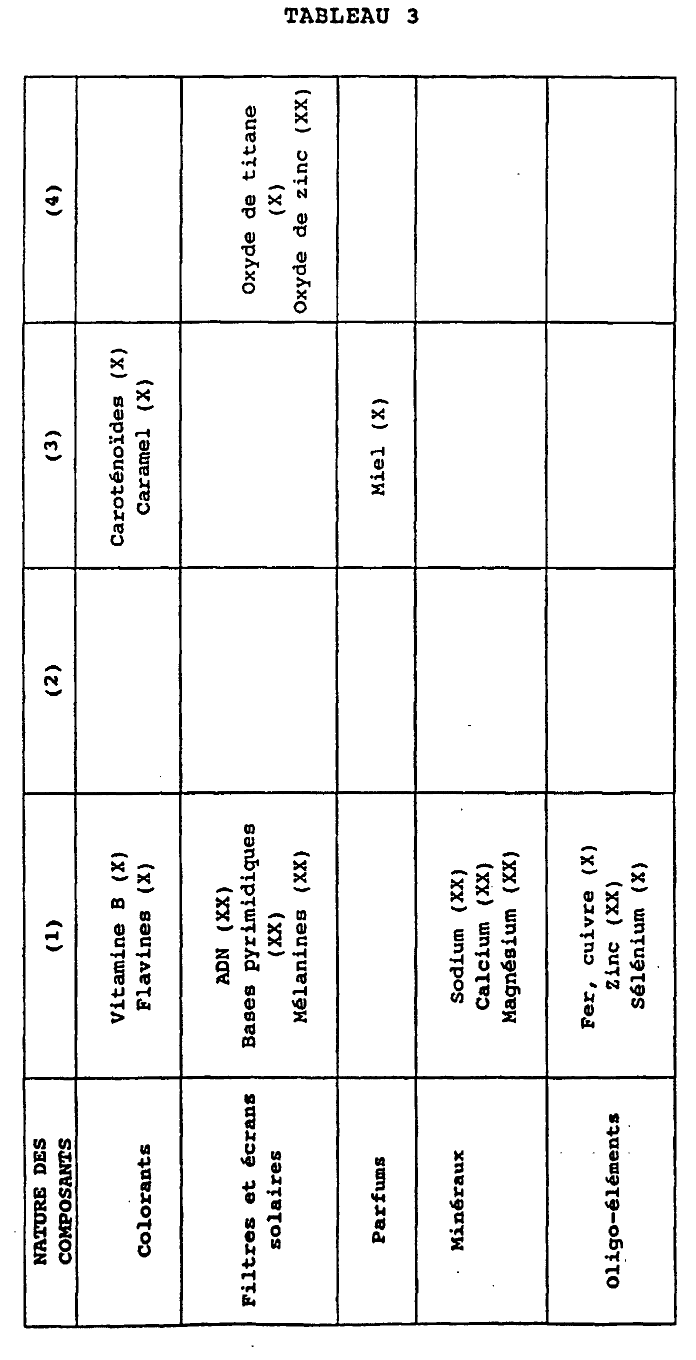

Les compositions de quelques produits cosmétiques selon l'invention sont données ci-après.

| **PRODUIT 1: Gel nutritif et hydratant** | |
|---|---|
| **PHASE A:** | |
| Composition nutritive | |
| (acides aminés, vitamines, oligo-éléments) | qsp 100 |
| Sel de sodium d'ADN | 2-5 % |
| Système de conservation | |
| (glucose/glucose oxydase/lactoperoxydase) | 0,9-1,1 % |
| | |

| **PHASE B:** | |
|---|---|
| Mucopolysaccharides | 20-30 % |
| Superoxyde dismutase | 0,5-1 % |
| Acide citrique | 0,2-0,5 % |
| Citrate trisodique | 0,5-2 % |

| **PRODUIT 2 : Spray rafraîchissant et apaisant** | |
|---|---|
| **PHASE A:** | |
| Composition nutritive (acides aminés, vitamines, oligo-éléments) | qsp 100 |
| Système de conservation | |
| (glucose/glucose oxydase/lactoperoxydase) | 0,9-1,1 % |
| | |

| **PHASE B:** | |
|---|---|
| Acide citrique | 0,2-0,5 % |
| Citrate trisodique | 0,5-2 % |
| | |

| **PHASE C:** | |
|---|---|
| Rhamnose | 0,01-5 % |

| **PRODUIT 3 : Crème (1) antiradicalaire pour peaux grasses** | |
|---|---|
| **PHASE A:** | |
| Acétate de tocophérol | 0,2-2 % |
| Acide stéarique | 3-5 % |
| Squalène | 2-7 % |
| Triglycérides | 2-7 % |
| | |

| **PHASE B:** | |
|---|---|
| Eau | qsp 100 |
| L-Arginine | 1-2 % |
| Glycérine | 1-2 % |
| Acide citrique | 0,2-0,5 % |
| Citrate trisodique | 0,5-2 % |
| | |

| **PHASE C**: | |
|---|---|
| Composition nutritive (acides aminés, vitamines, oligo-éléments) | 45-55 % |
| Système de conservation (glucose/glucose oxydase/lactoperoxydase) | 0,9-1,1 % |
| Superoxyde dismutase | 0,5-1 % |

| **PRODUIT 4 : Crème (2) hydratante pour peaux sèches** | |
|---|---|
| **PHASE A:** | |
| Acide oléique | 0,2-0,3 % |
| Acide palmitique | 0,2-0,3 % |
| Acide béhénique | 0,2-0,3 % |
| Acide stéarique | 0,1-0,2 % |
| Acide linoléique | 0,1-0,2 % |
| Acide arachidique | 0,05-0,1% |
| Triglycérides | 0,1-0,2 % |
| Cholestérol | 0,9-1 % |
| Cholestérol ester | 0,02-0,04% |
| Phospholipides | 1,5-2,5 % |
| Squalène | 3-7 % |
| | |

| **PHASE B:** | |
|---|---|
| Eau | qsp 100 |
| L-Arginine | 1-2 % |
| Acide citrique | 0,2-0,5 % |
| Citrate trisodique | 0,5-2 % |
| | |

| **PHASE C:** | |
|---|---|
| Composition nutritive (acides aminés, vitamines, oligo-éléments) | 45-55 % |
| Système de conservation (glucose/glucose oxydase/lactoperoxydase) | 0,9-1,1 % |
| | |

| **PHASE D:** | |
|---|---|
| Mucopolysaccharides | 1-3 % |

| **PRODUIT 5 : Lait démaquillant désensibilisant** | |
|---|---|
| **PHASE A:** | |
| Acide stéarique | 2-5 % |
| Squalène | 2-7 % |
| Triglycérides | 2-7 % |
| | |

| **PHASE B:** | |
|---|---|
| Eau | qsp 100 |
| L-Arginine | 1-2 % |
| Acide citrique | 0,2-0,5 % |
| Citrate trisodique | 0,5-2 % |
| | |

| **PHASE C:** | |
|---|---|
| Composition nutritive | |
| (acides aminés, vitamines, oligo-éléments) | 45-55 % |
| Superoxyde dismutase | 0,5-1 % |
| Système de conservation | 0,9-1,1 % |
| Fucose | 0,005- 1% |

| **PRODUIT 6 : Lotion hydratante** | |
|---|---|
| **PHASE A:** | |
| Eau | qsp 100 |
| L-sérine | 1-3 % |
| Glycérine | 1-2 % |
| Urée | 1-3 % |
| Acide citrique | 0,2-0,5 % |
| Citrate trisodique | 0,5-2 % |
| | |

| **PHASE B:** | |
|---|---|
| Système de conservation (glucose/glucose oxydase/lactoperoxydase) | 0,9-1,1 % |

| **PRODUIT 7 : Huile sèche** | |
|---|---|
| **PHASE A:** | |
| Squalène | 30-70 % |
| Triglycérides | 30-70 % |

| **PRODUIT 8 : Crème solaire bioprotectrice** | |
|---|---|
| **PHASE A:** | |
| Acétate de tocophérol | 0,2-2 % |
| Acide stéarique | 3-5 % |
| Squalène | 2-7 % |
| Triglycérides | 2-7 % |
| Oxydes de titane | 1-20 % |
| | |

| **PHASE B:** | |
|---|---|
| Eau | qsp 100 |
| L-Arginine | 1-2 % |
| Glycérine | 1-2 % |
| Acide citrique | 0,2-0,5 % |
| Citrate trisodique | 0,5-2 % |
| | |

| **PHASE C:** | |
|---|---|
| Système de conservation (glucose/glucose oxydase/lactoperoxydase) | 0,9-1,1 % |

| **PRODUIT 9: Lait nettoyant régulateur et anti-sensibilisant** | |
|---|---|
| **PHASE A:** | |
| Acide stéarique | 2-5 % |
| Squalène | 2-7 % |
| Triglycérides | 2-7 % |
| | |

| **PHASE B:** | |
|---|---|
| Eau | qsp 100 |
| L-Arginine | 1-2 % |
| Glycérine | 1-2 % |
| Acide citrique | 0,2-0,5 % |
| Citrate trisodique | 0,5-2 % |
| | |

| **PHASE C:** | |
|---|---|
| Système de conservation | |
| (glucose/glucose oxydase/lactoperoxydase) | 0,9-1,1 % |
| Phospholipides | 2-10 % |
| | |

| **PHASE D:** | |
|---|---|
| Rhamnose | 0,1-5% |
| Fucose | 0,0005-1% |

Les produits selon l'invention sont par exemple sous forme de lotion, de lait, de crème, de savon, etc..., en fonction de leur prescription ou utilisation. Ils sont sous une forme topique stable, monophasique, biphasique, ou triphasique, par exemple.

## Revendications

1. Produit cosmétique ou d'hygiène corporelle, ou dermo-thérapeutique, **caractérisé en ce qu'**il comprend pour au moins 98% en poids, uniquement des constituants biodermiques cytocompatibles avec la peau, exprimant ensemble de manière interactive au moins une bioactivité topique, identique ou différente de la bioactivité d'au moins un constituant biodermique, et conférant audit produit une composition sous forme galénique topique stable, excluant pratiquement tout excipient non biodermique autre que de l'eau.

2. Produit selon la revendication 1, **caractérisé en ce que** lesdits constituants biodermiques sont biomimétiques avec un composant de la peau, et ont chacun une teneur pondérale dans ledit produit, différente de celle du même composant dans la peau.

3. Produit selon la revendication 2, **caractérisé en ce qu'**un constituant biodermique et biomimétique, majoritaire, est un constituant constitutif de la peau, et un autre constituant biodermique et biomimétique, minoritaire, est un nutriment de la peau.

4. Produit selon la revendication 1, **caractérisé en ce que** chaque constituant biodermique est choisi parmi les espèces ou entités, biologiques, minérales, organiques, ou biochimiques, composant l'épiderme ou le derme.

5. Produit selon la revendication 1, **caractérisé en ce qu'**au moins un constituant biodermique est choisi parmi les nutriments de la peau.

6. Produit selon les revendications 4 et 5, **caractérisé en ce que** la composition est complétée par un constituant non biodermique mais cytocompatible avec la peau.

7. Produit selon la revendication 1, **caractérisé en ce qu'**il est sous forme biphasique, et par exemple constitué par une dispersion eau dans huile, ou huile dans eau.

8. Produit selon la revendication 1, **caractérisé en ce que** la totalité de sa composition est constituée par les constituants biodermiques cytocompatibles avec la peau.

## Patentansprüche

1. Kosmetik-, Körperhygiene- oder Hautheilmittelprodukt, **dadurch gekennzeichnet, daß** es zumindest 98 Gew.-% ausschließlich biodermale Bestandteile aufweist, die mit der Haut zytokompatibel sind, und die zusammen auf interaktive Weise zumindest eine solche topische Bioaktivität entfalten, die mit der Bioaktivität von zumindest einem biodermalen Bestandteil identisch oder von dieser verschieden ist, und die dem Produkt eine Zusammensetzung in stabiler topischer galenischer Form verleihen, wobei jeglicher andere nicht-biodermale Bestandteil, außer Wasser, praktisch ausgeschlossen ist.

2. Produkt nach Anspruch 1, **dadurch gekennzeichnet, daß** die biodermalen Bestandteile biomimetisch zu einem Hautbestandteil sind, wobei jeder Bestandteil in dem Produkt einen Gewichtsgehalt aufweist, der sich von dem des gleichen Bestandteils in der Haut unterscheidet.

3. Produkt nach Anspruch 2, **dadurch gekennzeichnet, daß** ein biodermaler und biomimetischer Hauptbestandteil ein Grundbestandteil der Haut ist, und ein anderer biodermaler und biomimetischer Nebenbestandteil ein Nährstoff der Haut ist.

4. Produkt nach Anspruch 1, **dadurch gekennzeichnet, daß** jeder biodermale Bestandteil ausgewählt ist aus den biologischen, mineralischen, organischen oder biochemischen Spezies oder Gebilden, aus denen die Epidermis oder die Dermis zusammengesetzt ist.

5. Produkt nach Anspruch 1, **dadurch gekennzeichnet, daß** zumindest ein biodermaler Bestandteil ausgewählt ist aus den Nährstoffen der Haut.

6. Produkt nach Anspruch 4 oder 5, **dadurch gekennzeichnet, daß** die Zusammensetzung durch einen nicht-biodermalen aber mit der Haut zytokompatiblen Bestandteil ergänzt ist.

7. Produkt nach Anspruch 1, **dadurch gekennzeichnet, daß** es in biphasischer Form vorliegt und beispielsweise aus einer Dispersion von Wasser in Öl oder von Öl in Wasser gebildet ist.

8. Produkt nach Anspruch 1, **dadurch gekennzeichnet, daß** die Gesamtheit seiner Zusammensetzung aus mit der Haut zytokompatiblen biodermalen Bestandteilen gebildet ist.

## Claims

1. Cosmetic or corporal hygiene or dermotherapeutic product, **characterized in that** it comprises, to at least 98% by weight, only biodermal constituents which are cytocompatible with the skin, which together interactively express at least one topical biological activity, which is identical to or different from the biological activity of at least one biodermal constituent, and which give the said product a composition in topical pharmaceutical form which is stable, excluding virtually every non-biodermal excipient other than water.

2. Product according to Claim 1, **characterized in that** the said biodermal constituents are biomimetic with a component of the skin and each have a weight content in the said product which is different from that of the same component in the skin.

3. Product according to Claim 2, **characterized in that** a major biodermal and biomimetic constituent is a constitutive constituent of the skin, and another, minor biodermal and biomimetic constituent is a skin nutrient.

4. Product according to Claim 1, **characterized in that** each biodermal constituent is chosen from the biological, mineral, organic or biochemical species or entities of which the epidermis or the dermis is composed.

5. Product according to Claim 1, **characterized in that** at least one biodermal constituent is chosen from skin nutrients.

6. Product according to Claims 4 and 5, **characterized in that** the composition is supplemented with a non-biodermal constituent which is cytocompatible with the skin.

7. Product according to Claim 1, **characterized in that** it is in two-phase form and, for example, consists of a water-in-oil or oil-in-water dispersion.

8. Product according to Claim 1, **characterized in that** its entire composition consists of the biodermal constituents which are cytocompatible with the skin.
